# EUROPEAN PATENT SPECIFICATION

(11) **EP 4 048 373 B1**
(45) Date of publication and mention of the grant of the patent: **18.06.2025**
(21) Application number: 20878166.6
(22) Date of filing: 03.12.2020
(51) Int. Cl.: A61N 1/32, A61K 9/00, A61N 1/04

(54) **CONTINUOUS OR PERIODICAL TRANSDERMAL DELIVERY OF EXOGENOUS MOLECULE**
KONTINUIERLICHE ODER PERIODISCHE TRANSDERMALE VERABREICHUNG EINES EXOGENEN MOLEKÜLS
ADMINISTRATION TRANSDERMIQUE CONTINUE OU PÉRIODIQUE DE MOLÉCULE EXOGÈNE

(43) Date of publication of application: 31.08.2022
(73) Proprietor: Kiffik Inc., Châteauguay, QC J6K 2N2 (CA)
(72) Inventor: ESSALIK, Abdeltif, Québec G6W 1Z8 (CA); BENSALEM, Samia, 4500 Solothurn (CH); LUO-ALT, Yahui, 90425 Nuremberg (DE)
(74) Representative: Hoffmann Eitle
(86) International application number: PCT/CA2020/051415
(87) International publication number: WO 2021/077221

(56) References cited:
- EP-A1- 2 398 385
- EP-B1- 2 424 602
- EP-B1- 2 674 188
- US-A- 5 019 034
- US-A1- 2011 112 520
- US-B2- 10 245 428
- US-B2- 9 986 979

## Description

### TECHNICAL FIELD

It is provided a device for delivering transdermally a molecule non-invasively to a subject comprising irreversibly electroporating a region of the skin.

### BACKGROUND

Delivery of drugs through the skin has always been a challenging area for research due to the barrier properties that exhibit the outermost layer of skin stratum corneum. In the last two decades, transdermal drug delivery systems have been developed and proven to offer significant clinical benefits over other dosage forms or formulations. Because transdermal drug delivery offers controlled as well as predetermined rate of release of a drug into the patient, it represents a mean able to maintain steady state blood and systemic concentration. Transdermal delivery avoids the stomach environment where the drug can be degraded and the first pass effect where active drug molecules can be converted to inactive molecules or even to molecules responsible for side effects. Transdermal drug delivery provides a mean to a steady concentration in plasma and/or the interstitial fluid and potentially the drug input can be stopped at any point after removal of the device allowing the delivery from the site. Developing means for delivering drug transdermally could also increase compliance and reduce medical costs, improve bioavailability, and represent a favorable route of administration for pediatric patients. It is also predicted that transdermal delivery can decrease chances of overdose (see Sachan and Bajpai, International Journal of Research and Development in Pharmacy and Life Sciences, December, 2013, 3(1): 748-765, Marwah et al., Drug Deliv, 2016, 23(2): 564-578).

Since the first transdermally delivered drug, Transderm Scop^{®}, a transdermal patch delivering scopolamine, introduced by Novatris in 1979, innovations in transdermal delivery systems can be classified according to three stages of development. A first generation of systems were developed consisting of patches to deliver drugs that can cross the skin at acceptable therapeutic levels. In almost all transdermal patch models, the drug is stored in a reservoir that is enclosed between two different membranes. The first membrane which is in contact with the outside consists of an impermeable membrane and which may have an adhesive layer at its ends to allow adhesion of the patch to the skin. The second membrane which is in contact with the skin consists of a semi-permeable membrane which can serve as a barrier limiting the rate of transfer of the drug. Some first-generation patch models contain a drug dissolved in a liquid or a gel-based reservoir. These patches are characterized by four layers: an impermeable backing membrane, a drug reservoir, a semipermeable membrane that can act as a barrier limiting the rate of transfer of the drug and an adhesive layer. Other first-generation patches are characterized by the presence of the drug to be delivered in a solid polymer matrix. In this same generation, three-layered patches can be noted, eliminating the semi-permeable membrane or two layers, incorporating the drug directly into the adhesive.

A second generation of systems were developed to promote the distribution of small molecules by increasing the skin's permeability and driving forces for transdermal transport. The second generation of transdermal delivery systems can be recognized as those that have begun to improve skin permeability by using chemical permeation enhancers (CPEs) to widen the spectrum of possibly transdermally applicable molecules. However, the methods used such as conventional chemical enhancers, non-cavitational ultrasound and iontophoresis have generally failed to balance the increased permeability of the stratum corenum and to protect the deeper tissues from damage caused by these same methods.

A third generation of transdermal delivery systems, mainly targeting the stratum corenum, is now in development. This approach enables almost complete disruption of the stratum carenum wall and thereby more effectively deliver molecules transdermally. Novel chemical enhancers, ultrasound, electroporation and more recently microneedles, causing thermal ablation and microdermabrasion have been shown to deliver macromolecules, including vaccines and therapeutic proteins, across the stratum corenum in human clinical trials.

There is thus still a need to be provided with means for transdermally deliver a drug or molecule to a patient.

EP 2674188B1 relates to a system and method for the creation of small holes or perforations or micropores in a biological membrane of the subject and the subsequent transdermal delivery of drugs or other permeants into the subject via the formed micropores.

US 9 986 979 B2 relates to a non-invasive and continuous biomedical detections and non-invasive and continuous monitoring methods and devices for extracting and analyzing interstitial fluid extracted non-invasively and continuously from the skin of a subject comprising non-invasively electroporating the skin using a non pulsed voltage in combination with a pulsed voltage and applying negative pressure.

### SUMMARY

The present invention is defined by the subject-matter of independent claim 1. Preferred embodiments are given by the subject-matter of dependent claims.

One aim of the present disclosure is to provide a method for delivering transdermally a molecule non-invasively to a subject comprising the steps of irreversibly electroporating a region of the skin without generating any heat; applying a pressure at or near the region of the skin being irreversibly electroporated, the combination of irreversibly electroporating the skin and periodically applying the pressure forming openings in the skin; and delivering the molecule through the openings.

In an embodiment, a non-pulsed voltage and a pulsed voltage are generated between at least a first moving or stationary electrode and a second stationary electrode for electroporating the skin.

In another embodiment, the pressure is periodically pulsed or continuous.

In a further embodiment, the pressure applied at or near the region of the skin being electroporated is or is not monitored by a pressure sensor.

In a further embodiment, the subject is an animal or a human.

In another embodiment, the molecule is a drug, an antibody, a DNA molecule, a RNA molecule, a virus, an enzyme or a cell.

In an additional embodiment, the molecule is scopolamine, nitroglycerin, clonidine, estradiol, fentanyl, nicotine, testosterone, lidocaine, epinephrine, estradiol, norethindrone, ethinylestradiol, oxybutynin, tetracaine, methylphenidate, selegiline, retigabine, rivastigmine, AB-1001, acyclovir, buprenorphine, fertility hormone, granisetron, heat-labile enterotoxin of E. coli., human growth hormone, influenza vaccine, insulin, ketoprofen, parathyroid hormone, sufentanil, and/or triamcinolone acetonide, catalase, peroxidase, dismutase or a combination of the last three enzymes.

In another embodiment, the method provided herein further comprises delivering the molecule through a transfer limiting membrane controlling the rate of administration of the molecule to the openings.

It is also provided that the molecule to be delivered is dissolved in an aqueous, or a neutral or a lipophilic solution containing also a CPE, or in a gel, or in a cream or an ointment containing also a CPE. The chemical permeation enhancers (CPEs) as encompassed herein provides a mean to maintain the opened region of the skin open.

It is also provided a device for non-invasively delivering transdermally a molecule to a subject comprising means for irreversibly electroporating the skin by generating a non-pulsed voltage and a pulsed voltage between at least one first moving or stationary electrode and a second stationary electrode; a vacuum pump for periodically providing a pressure above the skin; and a chamber comprising the molecule to be delivered.

In an embodiment, at least one first and second electrodes are contained in the chamber.

In a further embodiment, the chamber is a separate part from the first and second electrodes used for the electroporation.

In another embodiment, the device described herein further comprises a sensor or a plurality of sensors for analyzing the delivery rate of the molecule and/or the temperature of the subject.

In an embodiment, the sensor or a plurality of sensors further comprise a wired or wireless communication device.

In another embodiment, the device further comprises an adhesive means for fixing the device on the skin of the subject.

In an embodiment, the vacuum pump is a peristaltic pump, a diaphragm pump, or a piston pump.

In another embodiment, the pressure generated by the vacuum pump is from about 2kPa to about 98 kPa.

In an embodiment, the vacuum generated by the pump is pulsed or continuous.

In another embodiment, the device further comprises a transfer limiting membrane for controlling the rate of administration of the molecule.

### BRIEF DESCRIPTION OF THE DRAWINGS

Reference will now be made to the accompanying drawings, showing by way of illustration, a preferred embodiment thereof.
Fig. 1 illustrates an illustration of impedimetric sensor before and after the deposition of the sensing layer on the sensing electrode in accordance to an embodiment.
Fig. 2A illustrates a schematic representation of an electrical equivalent circuit between the sensing electrode and the reference electrode in accordance to an embodiment.
Fig. 2B illustrates a Nyquist plot obtained when an alternative voltage with a sinusoidal amplitude comprised between 2 to 60 mV is superimposed to the sensing electrode is maintained at a DC voltage corresponding to the rest voltage or the open circuit voltage of the electrochemical cell (impedimetric sensor) in agreement with the electrical equivalent circuit shown in Fig. 2A.
Fig. 3 illustrates a top view of the bottom of a chamber as described herein in accordance to an embodiment.
Fig. 4 illustrates a planar top view of the bottom of the chamber in accordance to an embodiment.
Fig. 5 illustrates a top view of the upper part of the chamber in accordance to an embodiment.
Fig. 6 illustrates a top view of the upper part of the chamber in accordance to an embodiment.
Fig. 7 illustrates an assembled view of one chamber in conjunction with an electronic controller box as encompassed herein in accordance to an embodiment.
Fig. 8 illustrates an assembled view of one chamber in conjunction with the electronic controller box in accordance to an embodiment.
Fig. 9 illustrates a top view of three chambers connected in series to the same pump inside the electronic controller box in accordance to an embodiment.
Fig. 10A is a picture of an electrode assembly in accordance to an embodiment.
Fig. 10B is a picture of the electrode assembly as seen in Fig. 10A pressed on the skin of a subject.
Fig. 10C is a picture demonstrating that the electrode assembly does not pierce or injure the skin of the subject.
Fig. 10D is a picture of the micro-opening of the skin of a subject after applying pulsed electrical field for several minutes in accordance to the present disclosure.

It will be noted that throughout the appended drawings, like features are identified by like reference numerals.

### DETAILED DESCRIPTION

It is provided a claimed device for delivering transdermally a molecule non-invasively to a subject comprising the means for irreversibly electroporating a region of the skin without generating any heat; applying for a short period of time a pressure at or near the region of the skin being irreversibly electroporated, the combination of irreversibly electroporating the skin and applying the pressure for a short period of time forming openings in the skin; and delivering the molecule through the openings.

Accordingly, it is described a method and a device to overcome the barrier of the epidermis and especially the stratum carenum. Once the breakdown of this barrier is realized by the method and the device proposed hereinafter, any kind of drug regardless of its size, regardless of its hydrophobic/hydrophilic/neutral/lipophilic character will pass through this barrier and reach the dermis and hence be available locally or to the systemic circulation in the target body.

Disclosed herein is a method which creates one or more electrophilic conduit through the stratum corneum. These openings are permanent and have a duration limited only by the duration of the treatment. Otherwise, as long as these openings or conduits through the stratum corneum are in contact with the drug to be transferred to the general circulation, they will remain open and close only after removing the contact between them and the drug in question.

The device and a method as described in the Canadian patent No. 2655017 can be used to irreversibly form one or a plurality of openings or conduits solely through the stratum corneum. The conduit(s) hence formed will be the area of interest where a target molecule(s) present in a chemical composition or standing alone will be placed to reach the systemic circulation through its transport in the interstitial fluid present in the dermis and underneath the dermis.

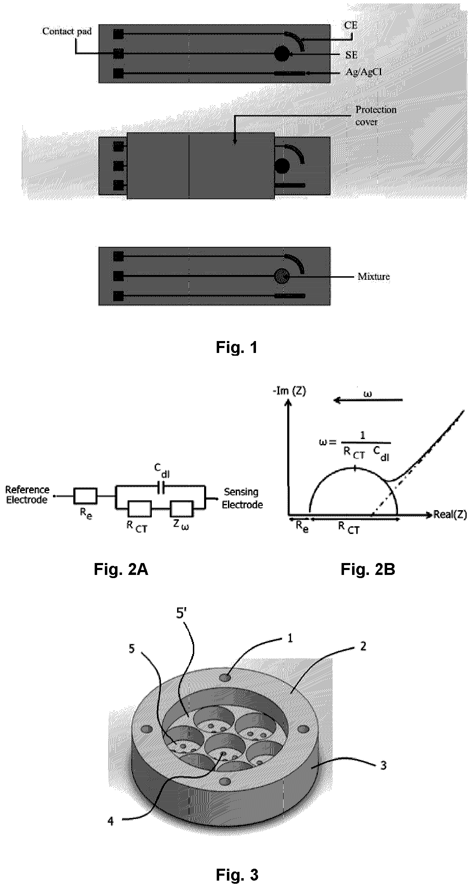

In an embodiment, the device used herein can comprise a sensing electrode as illustrated in Fig. 1. The electrode can comprise a stabilizer and a blocker of undesirable biomaterials, a promotor of adhesion such as aminosilane and nano-particles of an electronic conductor such as XC-72R on the surface of a carbon electrode (sensing electrode). The sensor electrode could be used with or without a counter-electrode.

It is described herein, measurement of an electrical parameter generated when a single frequency of an alternative voltage with a sinusoidal amplitude comprised between 2 to 60 mV is superimposed to the sensing electrode maintained at a DC voltage which corresponds to the rest voltage or the open circuit voltage of the electrochemical cell (impedimetric sensor). Such electrical parameters might include the variation of the values of the impedance (from Nyquist plot: Figs. 2a and 2b) or the phase angle/phase shift (from Bode plot) at the interface sensing electrode/interstitial fluid.

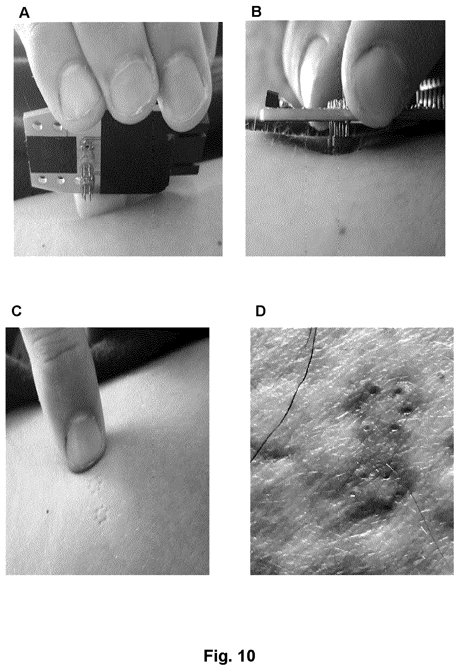

The electrical elements or network as described herein (see Fig. 2A and 10A) comprise resistance of the electrolyte between the reference electrode and the sensing electrode (Rₑ) which is a pure ohmic resistance, a double layer capacitance (C_{dl}), a charge transfer resistance (R_{ct}) which is not an ohmic resistance and a Warburg diffusion element (Z_{ω}). Z_{ω} is a constant phase element (CPE), with a constant phase of 45° (phase independent of frequency) and with a magnitude inversely proportional to the square root of the frequency.

The Nyquist plot obtained when an alternative voltage with a sinusoidal amplitude comprised between 2 to 60 mV is superimposed to the sensing electrode is maintained at a DC voltage corresponding to the rest voltage or the open circuit voltage of the electrochemical cell (impedimetric sensor) in agreement with the electrical equivalent circuit shown in Fig. 2A is seen in Fig. 2B. This plot is obtained when the frequency, ω, of the sinusoidal voltage has varied between 1 mHz to 100 KHz. The X axis is represented by the real values of the impedance (Real (Z)) and the Y axis is represented by the negative module of the imaginary values of the impedance (-lm (Z)) where Z is the impedance. In the plot in Fig. 2B, there is three distinct domains represented by an Ohmic resistance, Rₑ, a domain where the system is controlled by the charge transfer resistance, R_{ct}, at the interface extracted interstitial fluid and the sensing electrode with at least one time constant expressed by C_{dl}. In this disclosure, the value of the R_{ct} will increase with the increases, for example, of the concentration of the Covid-19 or its presence if the sensor is specific to the Covid-19.

Electroporation of the skin is affected by the amplitude of the applied voltage, the shape of the applied voltage and of the electrodes, the frequency of the applied signal, the intensity of the applied current and the duration of the applied signal. The amplitude of the applied voltage is expressed in volts and can vary from 5 V to 500 V depending on the above variables and it is now not independent on the distance between the positive and the negative electrodes if this distance is comprised between 1 to 10 cm. The frequency of the applied voltage may vary from 50 µS to 7000 µS. The shape of the signal is preferred to be square but other types of signal can be used. The preferred duration is less than three minutes depending on the above cited variables, but duration above the preferred value or under the preferred value is not excluded.

As encompassed herein, the term "irreversibly" is intended to describe real conduits formed through the stratum corneum. These conduits last as long as the target molecule is in contact with them. These conduits have real physical dimensions such as a diameter and a depth. They are note reversible.

The openings or conduits formed when the method described herein and is applied is intended to mean micro-holes through the stratum corneum formed following a short and controlled irreversible-electroporation as described herein (as seen in Fig. 10D). Irreversible-electroporation is the process by which and when applied on the skin of a living subject lead to the irreversible formation of micro-holes through the stratum corneum. The formed micro-holes last as long as they are needed.

More particularly, the device encompassed herein comprises means for electroporating the skin by generating a non-pulsed voltage and a pulsed voltage, and a sensor which are controlled electronically. The analysis of the signal obtained from the sensor in order to transform the information and to send the information to an alarm or a cell phone, for example, for further diffusion of the obtained information, is also controlled electronically. The irreversibly electroporation described herein of a region of the skin is done without generating any heat to preserve the connective tissue, in order to not denature molecules and collagen, eliminating any injuries to the cell scaffold and does not compromise the blood vessel matrix, which results in a clear margination of treated and nontreated areas.

As described in Canadian patent No. 2655017 the chamber for example used during the electroporation and sensing steps comprises electrodes, and more specifically, a reference electrode, a sensing electrode, and a counter electrode. Connection pads can be used to connect the electrodes to the electronic part of the device.

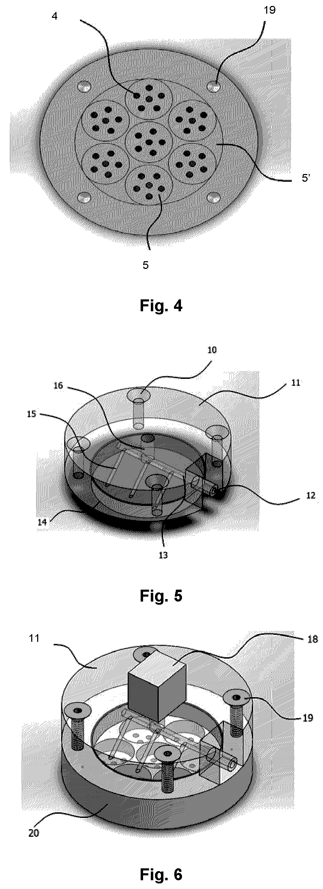

An embodiment of the chamber of the device encompassed herein is illustrated in Figs. 3 to 9. The device comprises an insulating body (see Fig. 3; **5****, 5',** **2** and **3**) maintained in contact with the skin through the epidermis using an adhesive or any other suitable sealing product or maintaining method **26.**

The chamber comprises at least one hole **1** to receive a screw **19,** a body **3** of the chamber is made of a polymer body **2** with holes **4,** wherein negative solid electrodes passes through said holes **4** to be in contact with the skin. A thin layer membrane **5** of 50 to 200 µm is used to keep the skin in place and to avoid its suction. A further thicker membrane **5'** of 2 to 5 mm is used to separate the different mini-chamber.

As seen in Fig. 5, in the upper part of the chamber, a polymer is used to make the upper part **11** of the chamber comprising the receiving ends **10** of holes that receive screws **19.** An outlet of the chamber **12** is connected to the inlet of the pump. In an embodiment, the device can comprise a collection micro-conduit **13** to collect the extracted native interstitial fluid from the micro-conduits **15** and serves, also, to accommodate the pressure sensor **16.** The connection between the bottom and the upper part of the chamber is made by the platform **14.**

The chamber **21** can be made completely from a polymeric material, a combination of a polymeric material or any other material that are electrically non-conductive or insulating.

As seen in Fig. 6, a cape **18** is used to protect a pressure sensor, wherein screws **19** to fix the bottom to the upper part **20** of the chamber.

The device can be covered by a material such as a capsule or an adhesive in order to facilitate its use, particularly in a situation where a liquid can surround the chamber **21.** The chamber **21** can include a sealed hole. Furthermore, in order to increase the fixture of the chamber **21** as described herein, a non-allergenic material such as a strap can be used to stabilize the contact between the device and the skin.

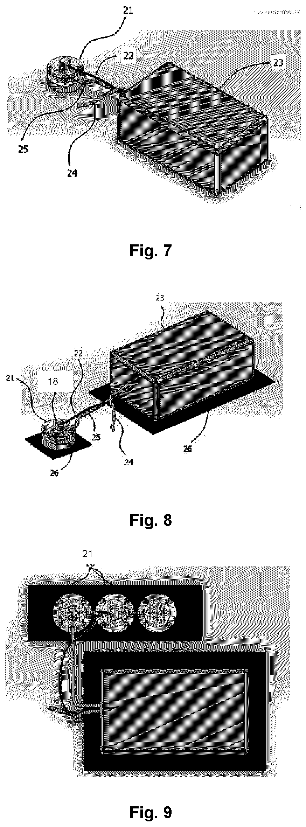

Fig. 7 illustrates in accordance to an embodiment an assembled view of one chamber **21** in conjunction with an electronic controller box **23.** The chamber **21,** is connected to the electronic controller box **23** containing the pump enclosed therein by an electrical cable **22** connecting the pressure sensor to the electronic controller box **23,** wherein an outlet **24** and inlet **25** of the pump enters said electronic controller box **23.**

Before the application of the chamber **21** on the skin, the skin can be gently cleaned by any known chemicals used in medicine to clean such skin or simply using a soap and/or water. Preferably, any chemicals that evaporate after cleaning are used such that they do not leave any residue at the surface of the skin. Moreover, the chemicals or the soap should not induce any allergenic reaction of the skin nor modify the structure of the skin. Furthermore, in some cases the use of Povidone-iodine (PVP-I), also known as iodopovidone must be used for skin disinfection before the electroporation and the installation of the chamber **21.** As soon as the skin is gently cleaned or preferably disinfected, the chamber **21** is attached on the top of the cleaned or disinfected skin by the help of the non-allergenic material such as the adhesive **26** or the strap or bracelet (Fig. 7). The adhesive **26** may or may not be based on an electrically conductive adhesive.

The molecule can be delivered for example through a micros conduit within the chamber **21.** The chamber **21** may or may not be isolated from a pump for example by a check valve or a one-way valve. The administration can be carried out by the application of a controlled pressure that is applied in the conduit system comprised namely of the chamber **21.** The controlled pressure is maintained by the action of the pump and the level of the pressure is continuously monitored and controlled using a pressure sensor or pressure switch, which could be located before or after the check valve.

The pressure is attained by activating the pump in an on/off fashion or in a continuously modulated fashion. The actual value of the pressure can be controlled using the pressure sensor or a pressure switch. The pump and the pressure sensor can be under the control of a microcontroller that shall continuously or discontinuously monitor the actual pressure in the device from reading the actual status of the pressure sensor or the pressure switch and shall accordingly activate or deactivate the operation of the pump in a continuous, discontinuous or modulated fashion in order to achieve the desired pressure. A temperature sensor can also be incorporated in order to improve the efficiency of the device. Furthermore, for example, a flow sensor could be installed between the outlet of the chamber **21** and the pump in order to inform the user that the administration process is going well.

The vacuum and overpressure are generated periodically inside the chamber **21** between the skin and the pump in order to improve the adhesion and the administration. Preferably, the vacuum pump can provide a vacuum that will provide enough suction to stretch the portion of the skin in the region from which the molecule is to be administered. As the suction provided by the vacuum pump is stretching the appropriate portion of the skin, the suction provided by the vacuum pump also causes the stretched portion. A vacuum pump that is suitable for the device defined herein can be a peristaltic pump, a diaphragm pump, a piston pump, a rotary vane pump, or any other pump that will perform the required functions set forth previously. Typically, the vacuum pump preferably employs a self-contained permanent magnet DC motor. Vacuum pumps that are suitable for this invention are well-known to those of ordinary skill in the art and are commercially available. The vacuum pump is preferably capable of providing a differential pressure down to about 0.90 atm, and is more preferably operated at from about 0.99 atm and 0.2 atm. The vacuum provided by the vacuum pump can be continuous or pulsed. It is preferred that the applied vacuum does not cause irreversible damage to the skin. It is preferred that the applied vacuum does not produce bruises and discolorations of the skin that persist for several weeks. It is also preferred that the level of applied vacuum and the duration of application of the vacuum do not be so excessive that it causes the dermis to separate from the epidermis, which results in the formation of a blister filled with fluid.

The irreversible electroporation of the skin is carried out by the application of a pulsed voltage between two electrodes or a plurality of electrodes forming a network of electrodes. As an illustration of the art herein, the excitation part of the process is carried out using at least two electrodes placed in contact with the exposed portion of the skin which is the negative electrode and the positive electrode which is used also as an adhesive. One of the excitation electrodes (negative electrode or electroporating electrode) is maintained in contact with the exposed portion of the skin during the nonheating irreversible electroporation.

Electroporation of the skin is affected by the amplitude of the applied voltage, the shape of the applied voltage and of the electrodes, the frequency of the applied signal and the duration of the applied signal. The amplitude of the applied voltage is expressed in volts and can vary from 0.100 V to 500 V depending on the above variables and the distance between the electrodes. The frequency of the applied voltage may vary from 100 µ-Second to 10 m-Second. The shape of the signal is preferred to be square but other type of signal can be used. The preferred duration is less than a second depending on the above cited variables, but duration above the preferred value or under the preferred value is not excluded.

Herein, irreversible electroporation is an event in which microsecond electrical pulses are applied on a living or non-living tissue, wherein the tissue is illustrated by the epidemies of a living subject, destabilizing the electrical potential across the tissue and resulting in irreversible nanoscale/microscale pores. Accordingly, to avoid the non-desirable Joule's effect or the generation of heat the electrical parameters are chosen such that the cell membrane is selectively targeted without inducing thermal damage to the rest of the tissue. These parameters are illustrated hereafter in Table 1.

**Table 1**

| Example of Electrical Parameters for two electrodes | |
|---|---|
| Parameters | Unit values |
| Applied pulsed voltage | 50 to 200 V |
| Applied DC voltage | 0.5 to 20 V |
| Distance between two negative electrodes | 1 to 100 mm |
| Frequency | 50 to 7000 µS |
| Current | 5 to 70000 µA |
| Duty cycle/pulse | 0.05 to 27 % |
| Duration | 60 to 1700 Seconds |
| Time between each 60 s of electroporation | 15 to 20 s |
| Measurement of the resistance | after each 60 Sec. between negative electrodes |

Due to its nonheating nature, the irreversible electroporation described herein does not affect the connective tissue nor does it denature molecules and collagen, eliminating any injuries to the cell scaffold and does not compromise the blood vessel matrix, and that the irreversible electroporation results in a clear margination of treated and nontreated areas.

Disclosed herein is a method that transfer an exogenous molecule or a plurality of molecules which have a therapeutic effect on the target subject or influence the wellbeing of the subject or have preventive actions on the subject, or have an improvement effect on the health of the subject. Herein the subject could be a human regardless of his age or an animal.

In an embodiment, the molecule or plurality of molecules having a therapeutic effect can be at least one of scopolamine, nitroglycerin, clonidine, estradiol, fentanyl, nicotine, testosterone, lidocaine, epinephrine, estradiol, norethidrone, ethinylestradiol, oxybutynin, tetracaine, methylphenidate, selegiline, rotigotine, rivastigmine, AB-1001, acyclovir, buprenorphine, fertility hormone, granisetron, heat-labile enterotoxin of *E*. *coli.,* human growth hormone, influenza vaccine, insulin, ketoprofen, parathyroid hormone, sufentanil, and/or triamcinolone acetonide, which are all drugs/molecules which have been administered by a transdermal mean.

Disclosed herein is an exogenous molecule or a plurality of molecules that could be used alone and could be a solid substance which has an excellent solubility in the interstitial fluid, could be a liquid which is preferably miscible in the interstitial fluid, could be in the form of a lyophilized molecule or could be in the form of a particle.

As encompassed herein, the exogenous molecule or plurality of molecules could have the molecular weight of a small ion such as lithium ion and could have a heavy weight such as a DNA or even heavy molecules such as a pathogen or a virus. Also encompassed is that the exogenous molecule or plurality of molecules described could be enclosed in a solid polymeric matrix, could be a part of an emulsion, could be dissolved in aqueous liquid, lipophilic liquid, could be enclosed in the form of an ointment based mixture, a gelatinous mixture, a solid solution or a powder mixture. The exogenous molecule or a plurality of molecules can also be dissolved before its use in the extracted interstitial fluid of the subject.

In an embodiment, it is disclosed that the method and devices encompassed herein include a transfer limiting membrane between the skin and the target molecule or a plurality of molecules to be transferred. This transfer limiting membrane will have solely the role to maintain a determined transfer rate of the target molecule or a plurality of molecules and may contain an enhancer which will have solely the role to maintain the micro-conduit(s) opened.

When the target molecule or the plurality of molecules are a biological substance such as an enzyme, a protein, an antibody for example, a stabilizer such as the human serum albumin will be added to the formulation of the substrate where the target molecule or the plurality of molecules will be encapsulated.

In an embodiment, the target molecule or plurality of molecules may include, as an example but not limited to, methylphenidate, superoxide dismutase or a mimetic of superoxide dismutase, catalase, amorphous insulin and testosterone.

In another embodiment, the method and devices described herein may include a miniature temperature sensor such as a miniature RTD, a miniature thermistor or a miniature thermocouple. The miniature temperature sensor will have solely the role to inform the user or to automatically put an end to the transfer of the exogenous molecule or the plurality of molecules to the dermis if an increase of temperature or sweat or a combination of both present a risk of an over dose.

While the disclosure has been described in connection with specific embodiments thereof, it will be understood that it is capable of further modifications and this application is intended to cover any variations, uses, or adaptations, including such departures from the present disclosure as come within known or customary practice within the art, and as may be applied to the essential features hereinbefore set forth.

## Claims

1. A device for non-invasively delivering transdermally a molecule to a subject comprising:
means for irreversibly electroporating the skin by generating a non-pulsed voltage and a pulsed voltage between at least one first moving or stationary electrode and a second stationary electrode;
a vacuum pump for providing a pressure above the skin; and
a chamber (21) comprising the molecule to be delivered.

2. The device of claim 1, wherein said at least one first and second electrodes are contained in the chamber (21).

3. The device of claim 2, wherein the chamber (21) is a separate part from the first and second electrodes used for the electroporation.

4. The device of any one of claims 1 to 3, further comprising a sensor or a plurality of sensors for analyzing the delivery rate of the molecule and/or the temperature of the subject.

5. The device of claim 4, wherein said sensor or a plurality of sensors further comprise a wired or wireless communication device.

6. The device of any one of claims 1 to 5, wherein said device further comprises an adhesive means (26) for fixing the device on the skin of the subject.

7. The device of any one of claims 1 to 6, wherein said vacuum pump is a peristaltic pump, a diaphragm pump, or a piston pump.

8. The device of any one of claims 1 to 7, wherein said pressure generated by the vacuum pump is from about 2kPa to about 98 kPa.

9. The device of any one of claims 1 to 8, wherein said vacuum generated by the pump is pulsed or continuous.

10. The device of any one of claims 1 to 9, further comprising a transfer limiting membrane for controlling the rate of administration the molecule.

11. The device of any one of claims 1 to 10, further comprising a pressure sensor configured to monitor the pressure applied at or near the region of the skin being electroporated.

12. The device of any one of claims 1 to 11, wherein the molecule is a drug, an antibody, a DNA molecule, a RNA molecule, a virus, or a cell; preferably the molecule is scopolamine, nitroglycerin, clonidine, estradiol, fentanyl, nicotine, testosterone, lidocaine, epinephrine, estradiol, norethidrone, ethinylestradiol, qxybutynin, tetracaine, methylphenidate, selegiline, rotigotine, rivastigmine, AB-1001, acyclovir, buprenorphine, fertility hormone, granisetron, heat-labile enterotoxin of *E*. *coli.,* human growth hormone, influenza vaccine, insulin, ketoprofen, parathyroid hormone, sufentanil, and/or triamcinolone acetonide.

## Patentansprüche

1. Vorrichtung zur nicht-invasiven transdermalen Verabreichung eines Moleküls an ein Subjekt, umfassend:
Mittel zur irreversiblen Elektroporation der Haut durch Erzeugen einer nichtpulsierenden Spannung und einer pulsierenden Spannung zwischen mindestens einer ersten sich bewegenden oder stationären Elektrode und einer zweiten stationären Elektrode;
eine Vakuumpumpe zum Bereitstellen eines Drucks über der Haut; und
eine Kammer (21), die das zu verabreichende Molekül umfasst.

2. Vorrichtung nach Anspruch 1, wobei die mindestens eine erste und zweite Elektrode in der Kammer (21) enthalten sind.

3. Vorrichtung nach Anspruch 2, wobei die Kammer (21) ein getrennter Teil von der ersten und zweiten Elektrode ist, die für die Elektroporation verwendet werden.

4. Vorrichtung nach einem der Ansprüche 1 bis 3, weiter umfassend einen Sensor oder eine Vielzahl von Sensoren zum Analysieren der Verabreichungsgeschwindigkeit des Moleküls und/oder der Temperatur des Subjekts.

5. Vorrichtung nach Anspruch 4, wobei der Sensor oder eine Vielzahl von Sensoren weiter eine drahtgebundene oder drahtlose Kommunikationsvorrichtung umfasst.

6. Vorrichtung nach einem der Ansprüche 1 bis 5, wobei die Vorrichtung weiter ein Anheftungsmittel (26) zum Befestigen der Vorrichtung auf der Haut des Subjekts umfasst.

7. Vorrichtung nach einem der Ansprüche 1 bis 6, wobei die Vakuumpumpe eine Schlauchpumpe, eine Membranpumpe oder eine Kolbenpumpe ist.

8. Vorrichtung nach einem der Ansprüche 1 bis 7, wobei der durch die Vakuumpumpe erzeugte Druck von etwa 2 kPa bis etwa 98 kPa beträgt.

9. Vorrichtung nach einem der Ansprüche 1 bis 8, wobei das der durch die Vakuumpumpe erzeugte Vakuum gepulst oder kontinuierlich ist.

10. Vorrichtung nach einem der Ansprüche 1 bis 9, weiter umfassend eine Transferbegrenzungsmembran zum Steuern der Geschwindigkeit der Verabreichung des Moleküls.

11. Vorrichtung nach einem der Ansprüche 1 bis 10, weiter umfassend einen Drucksensor, der so konfiguriert ist, dass er den an oder in der Nähe der elektroporierten Hautregion angewendeten Druck überwacht.

12. Vorrichtung nach einem der Ansprüche 1 bis 11, wobei das Molekül ein Medikament, ein Antikörper, ein DNA-Molekül, ein RNA-Molekül, ein Virus oder eine Zelle ist; wobei das Molekül bevorzugt Scopolamin, Nitroglycerin, Clonidin, Östradiol, Fentanyl, Niktoin, Testosteron, Lidokain, Epinephrin, Östradiol, Norethindron, Ethinylestradiol, Oxybutynin, Tetracain, Methylphenidat, Selegilin, Rotigotin, Rivastigmin, AB-1001, Acyclovir, Buprenorphin, Fruchtbarkeitshormon, Granisetron, wärmeunbeständiges Enterotoxin von E. *coli.,* menschlichem Wachstumshormon, Influenza-Impung, Isulin, Ketoprofen, Nebenschilddrüsenhormon, Sufentanil und/oder Triamcinolonacetonid ist.

## Revendications

1. Dispositif destiné à distribuer de manière non invasive par voie transdermique une molécule à un sujet comprenant :
un moyen pour une électroporation irréversible de la peau en générant une tension non pulsée et une tension pulsée entre au moins une première électrode mobile ou fixe et une seconde électrode fixe ;
une pompe à vide pour fournir une pression au-dessus de la peau ; et
une chambre (21) comprenant la molécule à distribuer.

2. Dispositif selon la revendication 1, dans lequel lesdites au moins une première et seconde électrodes sont contenues dans la chambre (21).

3. Dispositif selon la revendication 2, dans lequel la chambre (21) est une partie séparée des première et seconde électrodes utilisées pour l'électroporation.

4. Dispositif selon l'une quelconque des revendications 1 à 3, comprenant en outre un capteur ou une pluralité de capteurs pour analyser la vitesse de distribution de la molécule et/ou la température du sujet.

5. Dispositif selon la revendication 4, dans lequel ledit capteur ou une pluralité de capteurs comprennent en outre un dispositif de communication filaire ou sans fil.

6. Dispositif selon l'une quelconque des revendications 1 à 5, dans lequel ledit dispositif comprend en outre un moyen adhésif (26) pour fixer le dispositif sur la peau du sujet.

7. Dispositif selon l'une quelconque des revendications 1 à 6, dans lequel ladite pompe à vide est une pompe péristaltique, une pompe à diaphragme ou une pompe à piston.

8. Dispositif selon l'une quelconque des revendications 1 à 7, dans lequel ladite pression générée par la pompe à vide est d'environ 2 kPa à environ 98 kPa.

9. Dispositif selon l'une quelconque des revendications 1 à 8, dans lequel ledit vide généré par la pompe est pulsé ou continu.

10. Dispositif selon l'une quelconque des revendications 1 à 9, comprenant en outre une membrane de limitation de transfert pour commander la vitesse d'administration de la molécule.

11. Dispositif selon l'une quelconque des revendications 1 à 10, comprenant en outre un capteur de pression configuré pour surveiller la pression appliquée au niveau ou près de la région de la peau qui est électroporée.

12. Dispositif selon l'une quelconque des revendications 1 à 11, dans lequel la molécule est un médicament, un anticorps, une molécule d'ADN, une molécule d'ARN, un virus ou une cellule ; de préférence la molécule est la scopolamine, la nitroglycérine, la clonidine, l'œstradiol, le fentanyl, la nicotine, la testostérone, la lidocaïne, l'épinéphrine, l'œstradiol, la noréthidrone, l'éthinylœstradiol, l'oxybutynine, la tétracaïne, le méthylphénidate, la sélégiline, la rotigotine, la rivastigmine, l'AB-1001, l'acyclovir, la buprénorphine, l'hormone de la fertilité, le granisétron, l'entérotoxine thermolabile d'E. *coli.,* l'hormone de croissance humaine, le vaccin contre la grippe, l'insuline, le kétoprofène, l'hormone parathyroïdienne, le sufentanil et/ou l'acétonide de triamcinolone.
